# EUROPÄISCHE PATENTSCHRIFT

(11) **EP 1 848 814 B1**
(45) Veröffentlichungstag und Bekanntmachung des Hinweises auf die Patenterteilung: **29.06.2011**
(21) Anmeldenummer: 06708326.1
(22) Anmeldetag: 16.02.2006
(51) Int. Cl.: C12Q 1/37

(54) **SCREENINGVERFAHREN ZUR IDENTIFIZIERUNG PROTEASE-SEKRETIONSDEFIZIENTER MUTANTEN VON MIKROORGANISMEN**
SCREENING METHOD FOR IDENTIFYING PROTEASE SECRETION-DEFICIENT MUTANTS OF MICROORGANISMS
PROCEDE DE CRIBLAGE POUR IDENTIFIER DES MUTANTS DEFICIENTS EN SECRETION DE PROTEASE DANS DES MICRO-ORGANISMES

(30) Priorität: 16.02.2005 EP 05003272
(43) Veröffentlichungstag der Anmeldung: 31.10.2007
(73) Patentinhaber: Cilian AG, 48149 Münster (DE)
(72) Erfinder: HARTMANN, Marcus, 48147 Münster (DE); BROERMANN, André, 48145 Münster (DE)
(74) Vertreter: von Kreisler Selting Werner
(86) Internationale Anmeldenummer: PCT/EP2006/060034
(87) Internationale Veröffentlichungsnummer: WO 2006/087366

(56) Entgegenhaltungen:
- WO-A-99/14347
- US-A- 6 153 424
- US-A1- 2003 108 908
- US-B1- 6 566 122
- HÜNSELER P. ET AL.: "Isolation and characterization of a mutant of Tetrahymena thermophila blocked in secretion of lysosomal enzymes." JOURNAL OF CELL SCIENCE. AUG 1987, Bd. 88 ( Pt 1), August 1987 (1987-08), Seiten 47-55, XP009049673 ISSN: 0021-9533 in der Anmeldung erwähnt
- JONES E.W.: "TACKLING THE PROTEASE PROBLEM IN SACCHAROMYCES CEREVISIAE" METHODS IN ENZYMOLOGY, ACADEMIC PRESS INC, SAN DIEGO, CA, US, Bd. 194, 1991, Seiten 428-453, XP000943686 ISSN: 0076-6879
- HARTMANN M. ET AL.: "Screening for and characterization of phospholipase A1 hypersecretory mutants of Tetrahymena thermophila" APPLIED MICROBIOLOGY AND BIOTECHNOLOGY, SPRINGER VERLAG, BERLIN, DE, Bd. 54, Nr. 3, September 2000 (2000-09), Seiten 390-396, XP002208204 ISSN: 0175-7598 in der Anmeldung erwähnt
- PAECH C. ET AL.: "Zymogram of proteases made with developed film from nondenaturing polyacrylamide gels after electrophoresis" ANALYTICAL BIOCHEMISTRY, Bd. 208, Nr. 2, 1993, Seiten 249-254, XP002333535 ISSN: 0003-2697 in der Anmeldung erwähnt
- BRUNK C.F.: "Ciliates display promise for foreign gene expression" NATURE BIOTECHNOLOGY, NATURE PUBL., US, Bd. 17, Nr. 5, Mai 1999 (1999-05), Seiten 424-245, XP002956892 ISSN: 1087-0156

## Beschreibung

Gegenstand der Erfindung ist ein Verfahren zur Identifizierung Protease-sekretionsdefizienter Stämme von Mikroorganismen.

In der Biotechnologie besteht ein großes Bedürfnis nach Expressionsystemen, mit denen Proteine in großen Mengen und in der physiologisch vorkommenden Form produziert werden können. Eine von vielen Möglichkeiten ist die heterologe Expression in Mikroorganismen, die die Proteine produzieren, gegebenenfalls posttranslational modifizieren und dann sekretieren. Grundlage dafür ist es, den Mikroorganismus genetisch so zu verändern, dass das Fremdprotein hergestellt wird. Zu den Organismen, in denen eine heterologe Expression und anschließende Sekretion durchgeführt wird, zählen Ciliaten wie Tetrahymena und Hefen wie *Pichia pastoris.*

Die Nutzung solcher heterologer Expressionssysteme bietet viele Vorteile. So ist es möglich, *T. thermophila* kostengünstig und in kurzer Zeit zu hohen Zelldichten heranzuziehen (SALIBA et al., 1983; KIY & TIEDTKE, 1992). Ferner sind Methoden etabliert, den Organismus gentechnisch zu verändern, wodurch Fremdproteine in großen Mengen produziert werden können (TONDRAVI & YAO, 1986; YU et al., 1990; GAERTIG & GOROVSKY, 1992; GAERTIG et al., 1994; CASSIDY-HANLEY et al.; 1997). Diese Proteine werden in ähnlicher Form glycosysiert wie humane Proteine, was sie interessant für einen pharmazeutischen Einsatz macht (TANIGUCHI et al.; 1985). Einer der wesentlichen Vorteile dieses Expressionssystems besteht darin, dass von T. *thermophila* synthetisierte Proteine ins Medium sekretiert werden, was die Aufreinigung der Proteine vereinfacht (KIY, 1993).

Ein Problem bei der Verwendung solcher heterologer Expressionssysteme ist jedoch häufig die natürliche Sekretion von Proteasen. So ist beispielsweise bekannt, dass *T. thermophila* natürlicherweise große Mengen an Proteasen ins Medium (BANNO & NOZAWA, 1982; Banno et al., 1982; Banno et al., 1983) sekretiert. Da diese Proteasen nicht charakterisiert sind, liegen kaum nähere Informationen vor. Heterolog exprimierte Proteine sind im Vergleich zu endogenen Proteinen deutlich anfälliger gegenüber proteolytischem Abbau. Hingegen wird die funktionelle Aktivität der extrazellulären Enzyme von *T*. *thermophila* durch die sekretierten Proteasen im Wesentlichen nicht eingeschränkt (KIY, 1993b). Die Folge der proteolytischen Aktivität ist eine signifikante Verringerung der Ausbeute des heterolog expremierten Proteins.

Um die Degradation zu verhindern, sind verschiedene Ansätze möglich, wie die Zugabe von Proteaseinhibitoren oder von konkurrierenden Substraten wie Casein, oder die Erzeugung von Stämmen mit geringerer proteolytischer Aktivität. Zur Zeit stehen jedoch zum Beispiel für *Tetrahymena thermophila* keine Protease-sekretionsdefizienten Stämme zur Verfügung. Es wurde bereits eine Mutante beschrieben (HÜNSELER et al.; 1987), die generell in ihrer Sekretion eingeschränkt ist. Eine solche Mutante ist allerdings zur heterologen Expression von Proteinen ungeeignet, da zwar keine Proteasen sekretiert werden, jedoch auch die gewünschten Zielproteine nicht.

Die Ermittlung der sekretionsdefizienten Mutante erfolgte nach HÜNSELER et al., 1992 mit einem Screeningverfahren. Dabei wurden in eine Agarplatte Löcher gestanzt, die mit einer Pipette abgesaugt werden. In diese Löcher werden dann Zellkulturen gegeben. Das Verfahren ist relativ umständlich und nicht automatisierbar. Es besteht auch die Gefahr von Kreuzkontimanitionen bei Beschädigung der dünnes Agarschicht. Das Verfahren lässt sich auch nicht in großem Maßstab verwenden oder automatisieren.

Allgemein besteht für sekretierende Expressionssysteme ein hohes Bedürfnis nach Stämmen mit geringerer oder überhaupt keiner proteolytischer Aktivität.

Ein Verfahren zum Screening nach hypersekretorischen Mutanten von *T*. *thermophila* wurde bereits entwickelt (HARTMANN, 2000). Bei dieser Methode werden die Mutanten in 96-well Mikrotiterplatten angezogen. Die Zellen werden dann durch Ultrazentrifugation in eine Zone unterschiedlicher Dichte abgetrennt. Dabei wird jede der Kulturen mit einem Polymer (Ficoll) unterschichtet und die Zellen werden in das Ficoll zentrifugiert. Der Überstand wurde mit einer Pipette abgenommen. In Enzymtests wurden Mutanten bestimmt, die erhöhte Aktivität an sekretierten Proteinen wie β-Hexaminidase und saurer Phosphatase aufwiesen.

Dieses Verfahren erfordert einen hohen zeitlichen Aufwand. Ferner kann nicht ausgeschlossen werden, dass es zu einer Zelllyse kommt, bei der nicht sekretierte Proteine freigesetzt werden, die das Ergebnis verändern.

Der Erfindung liegt die Aufgabe zugrunde, ein Verfahren zum Screening nach verbesserten Sekretionssysteme zur heterologen Expression von Proteinen bereitzustellen. Insbesondere soll das Verfahren das Auffinden von Mutanten von Mikroorganismen ermöglichen, die eine verbesserte Ausbeute an sekretierten heterolog exprimierten Proteinen aufweisen.

Das Verfahren soll es ermöglichen, in kurzer Zeit und zu moderaten Kosten eine Vielzahl von Mutanten zu testen.

Die der Erfindung zugrunde liegende Aufgabe wird überraschenderweise gelöst durch ein Verfahren und Mikroorganismen nach einem der Ansprüche 1 bis 15.

Allgemein kann das Verfahren durchgeführt werden mit Mutanten von Mikroorganismen, die Proteasen sekretieren und zur Sekretion heterolog expremierter Zielproteine geeignet sind. Mit dem Begriff Sekretionsproteasen sind solche Proteasen gemeint, die der Wildtyp des Mikroorganismus in seine Umgebung sekretiert. Das erfindungsgemäße Verfahren ermöglicht die Messung der Proteaseaktivität *in vivo.*

Der Mikroorganismus ist vorzugsweise ausgewählt aus der Gruppe bestehend aus Ciliaten und Hefen. Solche Mikroorganismen expremieren eigene Proteine mit enzymatischer Aktivität wie Proteasen. Besonders geeignete Hefen sind *Pichia pastoris.* Das Verfahren eignet sich auch zum Screening einzelliger Algen, insbesondere *Chlamydomonas, Ulva* und *Euglena,* und Prokaryonten, insbesondere *Bacillus* und *Escherichia.* Besonders geeignete Ciliaten sind solche der Gattung *Tetrahymena,* insbesondere *Tetrahymena thermophila, malaccensis, elliotti, alphaeliotti, pyriformis, setosa, alphapyriformis, betapyriformis, leucophyris, silvani, vorax, tropicalis, alphatropicalis, betatropicalis, gammatropicalis, deltatropicalis, borealis, canadensis, rostrata, alphacanadensis, betacanadensis, mimbres, americanis americanis, paraamericanis, australis, hegewischi, hyperangularis, nanneyi, nippisingi, pigmentosa, pigmentosa oriasi, pigmentosa europigmentosa, sonneborni, asiatica, capricornis, patula, allensae, cosmopolitanis* oder *shanghaiensis.*

Die Mutanten der Mikroorganismen können durch bekannte Methoden wie Zufallsmutagenese oder gezielte Mutagenese erzeugt werden. Das Verfahren ist insbesondere vorteilhaft bei Mikroorganismen, bei denen Proteasesekretions-defiziente Stämme aus technischen Gründen oder aufgrund des Fehlens von genetischer Information nicht oder nur schwer erhältlich sind. Besonders bevorzugt sind kreuzungsgenetische Verfahren.

Eine besonders bevorzugtes Verfahren zur Erzeugung der Mutanten ist Uniparentale Cytogamie (UPC). Es handelt sich um ein kreuzungsgenetisches Verfahren. Dieses wird vorzugsweise durchgeführt wie von Cole und Bruns (1992) beschrieben. Auf das dort beschriebene Verfahren wird hiermit ausdrücklich Bezug genommen. Dieses Verfahren lässt bis zu 20fach mehr Mutationen expressiv werden, als beispielsweise das von Hünseler et al. (1987) verwendete Short-Circuit Genom Ausschluss-Verfahren. Dadurch ist die Mutantenausbeute um den Faktor 20 höher.

Die Inkubation der Mutante des Mikroorganismus mit dem Gel erfolgt vorzugsweise in einem geeigneten wässrigen Medium, vorzugsweise einem Nährmedium. In jedem Falle sollten Bedingungen (pH-Wert, Ionenkonzentration etc.) herrschen, die physiologischen Bedingungen ähneln, so dass das Absterben und die Lyse der Mikroorganismen verhindert wird.

Die Inkubation der Mikroorganismen mit dem Gel erfolgt in bevorzugten Ausführungsformen für 2 h bis 2 Wochen, insbesondere 1 bis 5 Tage.

Geeignete Nährmedien sind bzw. enthalten Magermilchpulver, Proteose Peptone, Hefeextrakt, Sojabohnen-Peptone, Ferrous Sulphate/Chelate Solution (100X, Fa. Sigma) und/oder Glucosemonohydrat. Es kann auch ein chemisch definiertes Medium (CDM) nach einer genauen Rezeptur der einzelnen Inhaltsstoffe verwendet werden.

Als Gel wird in bevorzugten Ausführungsformen ein Gelatine-, Agarose-, Agar- und/oder Polyacrylamidgel verwendet. Die Porengröße bzw. der Vernetzungsgrad wird so ausgewählt, dass sekretierte Proteine wie Proteasen in das Gel diffundieren können, während die Mikroorganismen im wesentlichen vom Gel ausgeschlossen sind.

In bevorzugten Ausführungsformen ist das Substrat für die Protease ausgewählt aus der Gruppe bestehend aus Casein, Derivaten des Casein, fluoreszent markiertem Casein und/oder Gelatine. Besonders geeignet sind BODIPY FL Casein und BODIPY TR-X Casein (Molecular Probes Inc., USA) oder ähnlich fluoreszent markierte Peptide oder Proteine.

Die Inkubation erfolgt vorzugsweise unter Bedingungen, bei denen der Mikroorganismus Proteasen sekretiert und sekretierte Proteasen in das Gel diffundieren können.

Das Abtrennen des Mikroorganismus von dem Gel erfolgt vorzugsweise durch abgießen, abpipettieren, absaugen und/oder abwaschen.

In einer bevorzugten Ausführungsform erzeugt die Proteolyse des Substrats ein Fluoreszenz- oder Absorptionssignal, dessen Intensität gemessen wird und anhand des Ergebnisses die Proteaseaktivität bestimmt wird.

Das erfindungsgemäße Verfahren wird unter Verwendung einer Mikrotiterplatte durchgeführt. In den Vertiefungen (Wells) der Platte wird jeweils ein Gel erzeugt. Dies erfolgt üblicherweise durch Eingießen einer Lösung, zum Beispiel einer Agaroselösung, in die Vertiefungen und anschließende Abkühlung. Bevorzugt sind übliche Mikrotiterplatten mit 96 Vertiefungen. Das Verfahren der Erfindung kann automatisiert und/oder unter Verwendung einer Vielzahl von zu testenden Mutanten durchgeführt werden. In jede Vertiefung der Mikrotiterplatte wird eine Mutante gegeben. Das muss nicht bedeuten, dass nur eine einzige Zelle zugegeben wird. Es kann auch eine Vielzahl von Zellen zugegeben werden mit identischer DNA. Eine solche Vielzahl von Zellen wird erzeugt, indem diese sofern vereinzelt und danach vermehrt wurde.

Das Verfahren der Erfindung ermöglicht das gezielte Screening nach Mutanten, die spezifisch keine oder weniger aktive Proteasen ins Medium sekretieren, während die Sekretion anderer Proteine und zu mindestens der heterolog exprimierten Proteine vorzugsweise nicht oder nur geringfügig eingeschränkt ist. Dabei ist für die Funktionsweise des Assays unerheblich, ob die Proteasen in inaktiver Form oder überhaupt nicht sekretiert werden. Entscheiden ist, dass die verminderte Proteaseaktivität eine Steigerung in der Ausbeute des Zielproteins zur Folge hat.

Verfahren zur Generierung von Mutanten von Ciliaten über Zufallsmutagenese sind bereits etabliert (CRUEGER & CRUEGER, 1989). Ebenso sind kreuzungsgenetische Manöver beschrieben, mit deren Hilfe eine Mutation zur Expression gebracht werden kann (COLE & BRUNS, 1989). Diese Verfahren sind aufgrund des Nuklear-Dimorphismuses bei *T*. *thermophila* notwendig. Ferner ist es möglich mit Hilfe einer Verteil-Apparatur und der Poisson-Lotterie die Mutanten auf 96-well Mikrotiterplatten zu verteilen.

Das erfindungsgemäße Verfahren weist zahlreiche Vorteile gegenüber bekannten Verfahren auf:

Das Verfahren ist spezifisch, da nicht Mutanten ermittelt werden, deren Sekretionssystem allgemein beeinträchtigt ist und die daher nicht zur heterologen Sekretion von anderen Proteinen geeignet wären.

Es wird ausschließlich durch die Aktivität der sekretierten Proteasen im Medium ein Signal erzeugt. Wegen den schonenden Assaybedingungen kommt es während des Screenings zu keiner Zelllyse und somit nicht zu einer Verfälschung des Ergebnisses durch intrazelluläre Proteasen.

Es können verschiedene unspezifische Protease-Substrate eingesetzt werden. Dies ist vorteilhaft, da über die Substratspezifität sekretierter Proteasen zum Beispiel bei Ciliaten wenig bekannt ist. Somit liefert das Screening Ergebnisse, die die gesamte Proteaseaktivität betreffen.

Das erfindungsgemäße Verfahren ist einfach und schnell durchführbar und erlaubt die Untersuchung von Ciliatenkulturen in ihrem Nährmedium. Dadurch entfallen Aufreinigungsschritte, wie die Zentrifugation bei dem Verfahren nach (Hartmann et al., 2000). Das einfache Verfahren ermöglicht es, große Mengen an Mutanten beispielsweise im 96-well Maßstab auf die Aktivität extrazellulärer Proteasen zu testen.

Insbesondere bei Erzeugung einer hohen Anzahl von Mutanten durch Uniparentale Cytogamie (UPC) wird eine sehr hohe Effizienz erreicht.

Das Verfahren liefert Mutanten, die anschließend bezüglich ihrer Sekretionseigenschaften genauer analysiert werden können. Die erhaltenen Stämme können dann als heterologe Expressionssysteme genutzt werden.

Das Verfahren macht sich die Diffusion expremierter Proteasen in ein Gel zunutze. Verfahren zum Auffinden von Proteasen, bei denen die Proteasen in ein Gel transferiert werden, waren bereits nach dem Stand der Technik bekannt, beispielsweise Paech et al., 1993. Es handelt sich dabei um Verfahren, bei denen die Proteasen zum einen vor dem Eintreten in das Gel aufgereinigt werden, zum anderen im Gel elektrophoretisch aufgetrennt werden. Das erfindungsgemäße Verfahren ist völlig unterschiedlich, da keine Aufreinigung der Proteasen erforderlich ist und da keine Elektrophorese und Anlegen einer elektrischen Spannung erfolgt.

Ein erfindungsgemäßes Screeningverfahren kann beispielsweise wie folgt durchgeführt werden:

Die Mutanten werden in den Vertiefungen einer 96-well Mikrotiterplatte angezogen. Die Kulturen befinden sich in flüssigem Nährmedium, welches sich auf einer Schicht aus Agarose befindet. Während der Inkubation diffundieren die extrazellulären Enzyme, unter anderem auch die Proteasen, zum Teil in die Agarose. Nachdem das Nährmedium mit den Zellen ausgeschlagen wurde, wird ein Casein-Derivat auf die Agarose gegeben, welches ebenfalls zum Teil in die Agarose diffundiert. Wird dieses Casein-Derivat durch proteolytische Aktivität gespalten, wird ein Fluoreszenzsignal erzeugt, welches proportional zur Proteaseaktivität ist. Die Intensität des Signals kann beispielsweise mit Hilfe eines Mikrotiterplatten-Fluoreszenz-Readers im Rahmen einer Endpunktbestimmung oder durch die Aufnahme einer Kinetik detektiert werden. Dabei ist ein geringes Fluoreszenzsignal ein Indiz für einen Protease-sekretionsdefizienten Stamm.

In einer weiteren Ausführungsform kann ein erfindungsgemäßes Screeningverfahren wie folgt durchgeführt werden:

Die bereits einige Zeit in Mikrotiterplatten inkubierten Kulturen werden mittels einer 8-Kanalpipette auf die mit Gelatine beschichtete Seite eines voll belichteten und entwickelten Röntgenfilms punktförmig aufgetragen. Die Tropfen bestehen aus Nährmedium, in welches bereits während der Inkubation die extrazellulären Enzyme sekretiert wurden. Ferner befinden sich die Zellen in dem Medium. Es ist darauf zu achten, dass die Tropfen nicht austrocknen, um eine Zellyse zu verhindern. Die Tropfen verbleiben einige Zeit auf der Gelatine, so dass diese von den vorhandenen Proteasen hydrolysiert werden kann. Werden die Tropfen dann vom Film gespült und der Film anschließend in einen speziellen Puffer gelegt, entstehen an den Stellen, an denen die Gelatine hydrolysiert wurde, Klärhöfe. Ein solcher Klärhof ist somit ein Indiz für hohe Aktivität extrazellulärer Proteasen.

Die erfindungsgemäßen Verfahren zeichnen sich dadurch aus, dass selektiv die Aktivität der Proteasen im Überstand detektiert wird, ohne dass aufwendige Verfahren zur Gewinnung eines zellfreien Überstandes nötig sind. Ferner ist es möglich, die Stämme auf die Aktivität von Proteasen zu testen, die nicht näher charakterisiert sind, da unspezifische Substrate (Casein-derivate, Gelatine) zum Einsatz kommen. Beide Verfahren zeichnen sich durch schnelle Durchführbarkeit und geringe Kosten aus, so dass eine hohe Zahl an Mutanten getestet werden kann.

### Ausführbeispiele:

### Verfahren 1:

Die Mutationen wurden durch chemische Zufallsmutagenese erzeugt und anschließend durch das kreuzungsgenetische Verfahren der Uniparentalen Cytogamie (kurz: UPC) gemäß COLE und BRUNS, 1992 zur Expression gebracht. Somit wurden Mutanten generiert. Nach Herstellung der Mutanten wurden Mikrotiterplatten für das Screening wie folgt hergestellt:

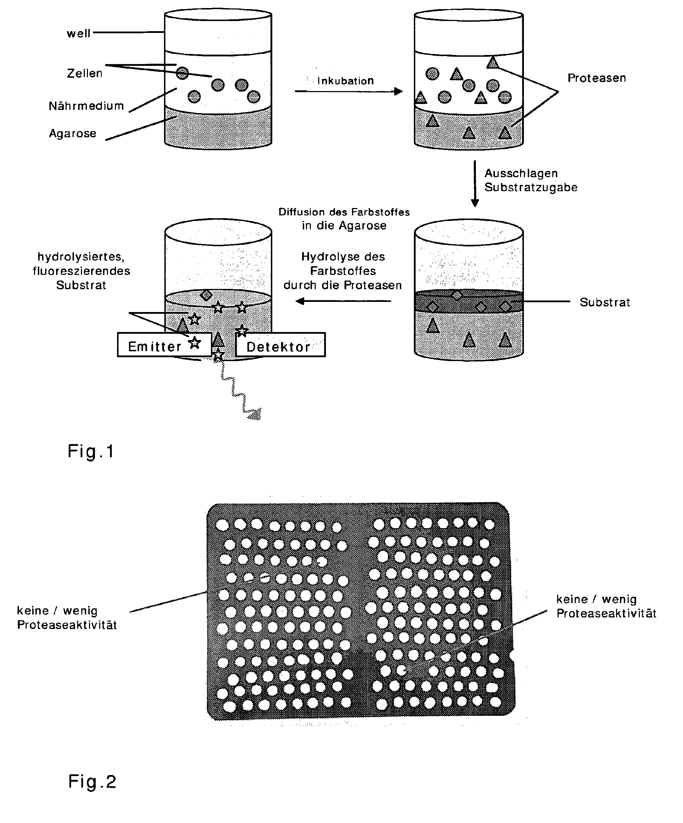

In die Vertiefungen (Wells) einer 96-well Mikrotiterplatte wird steril Agarose (1%; w/v) gegossen. Nach Erstarren der Agarose wird diese mit Nährmedium überschichtet. Das Animpfen der Vertiefungen mit den mutagenisierten Stämmen von *Tetrahymena* erfolgt mittels eines selbst hergestellten 96er Stempels, ausgehend von zuvor angelegten Mikrotiterplatten, in denen die Klone angezogen wurden. Nach drei Tagen Inkubation bei 30 °C wird das Nährmedium mit den Zellen ausgeschlagen (abgegossen) und die Vertiefungen werden zweimal mit Puffer (Tris-HCl; 50 mM; pH 7,4) gewaschen. In die Vertiefungen werden jeweils 100 µl Fluoreszenzfarbstoff als Substrat für die Enzymreaktion gegeben. Als Substrat kommen solche aus dem EnzChek Protease Assay-Kit E-6638 (Molecular Probes Inc. USA) zum Einsatz. Dabei wird der Farbstoff nach den Angaben des Herstellers verwendet, wobei die Gebrauchslösung 1 zu 2 durch den mitgelieferten Puffer verdünnt wird. Durch die Verdünnung kann die Probenanzahl deutlich erhöht werden, ohne dass die Sensitivität des Tests beeinträchtigt wird (Figur. 1).

Zur Detektion der Fluoreszenzintensität wurde der Reader FLx800 (BIO-TEK INSTRUMENTS, INC.) mit einem auf die Anregungs- und Detektionswellenlänge angepassten Filterpaar verwendet. Als Maß für die Proteaseaktivität kann direkt nach Zugabe des Substrates eine Kinetik aufgenommen werden, wobei der Grad der Steigung proportional zum Fluoreszenzsignal und somit auch zur Proteaseaktivität ist.

Alternativ ist es möglich nach einer Stunde Inkubation bei Raumtemperatur eine Endpunktsmessung durchzuführen. Die so gewonnenen Absolutwerte. spiegeln ebenfalls die Fluoreszenzintensität bzw. die Proteaseaktivität wieder.

### Verfahren 2:

Die Mutationen wurden durch chemische Zufallsmutagenese erzeugt und anschließend durch das kreuzungsgenetische Verfahren der Uniparentalen Cytogamie (kurz: UPC) gemäß COLE und BRUNS, 1992 zur Expression gebracht. Somit wurden Mutanten generiert. Nach Herstellung der Mutanten wurden Mikrotiterplatten für das Screening wie folgt hergestellt:

Mit Gelatine beschichtete Röntgenfilme (Ferrania) werden belichtet, entwickelt und fixiert. Vor dem Gebrauch werden die Filme für 10 min. mit Puffer (TrisHCl; 50 mM; pH 7,5) befeuchtet. Nach dieser Vorbehandlung müssen die Reste des Puffers mit Filterpapier sorgfältig entfernt werden. Auf die Gelatine beschichtete Seite werden 30 µl drei Tage alter Kulturen von Tetrahymena mittels einer 8-Kanal-Pipette aufgetragen. Es ist darauf zu achten, dass die Tropfen nicht ineinander verlaufen. Während der einstündigen Inkubation der Filme mit den Tropfen bei 30°C hydrolysieren die im Medium vorhandenen Proteasen die Gelatine, wobei die Zellen innerhalb des Tropfens überleben. Nach Ablauf der Inkubationszeit werden die Tropfen mit destilliertem Wasser von den Filmen gespült. Durch eine weitere einminütige Inkubation des Filmes in Glycin Puffer (0,1 M; pH 10,0; 50°C) sind an den Stellen, an denen die Gelatine abgebaut wurde Klärhöfe zu erkennen. Ohne die Gelatineschicht wird die die Schwarzfärbung verursachende Silberverbindung ausgewaschen. Ob es zu einer Auswaschung kommt oder nicht hängt somit von der Höhe der Aktivität der sekretierten Proteasen ab. Ebenso wird der Abbau der Gelatine durch die Parameter Inkubationszeit und Inkubationstemperatur beeinflusst, so dass der Test an die jeweils vorherrschenden Bedingungen angepasst werden muss.

Mit Hilfe dieser neuen Screening-Verfahren konnte eine Mutante von Tetrahymena isoliert werden, die deutlich weniger aktive Proteasen ins Nährmedium sekretiert bei im wesentlichen unverminderter Sekretion anderer Enzyme wie β-Hexaminidase und saurer Phosphatase.

Somit lassen sich mit Hilfe des neuen Screening-Verfahrens Mutanten herstellen, die ausschließlich für Proteasen sekretions-defizient sind, nicht aber generell in ihrer Fähigkeit eingeschränkt sind, Proteine in das sie umgebende Kulturmedium abzugeben. Deutlich wird dies, wenn die Sekretionskinetiken einer typischen Mutante, die mit dem neuen Screening-Verfahren gewonnen wurden, mit den Sekretionskinetiken von Wildtyp-Stämmen verglichen werden.

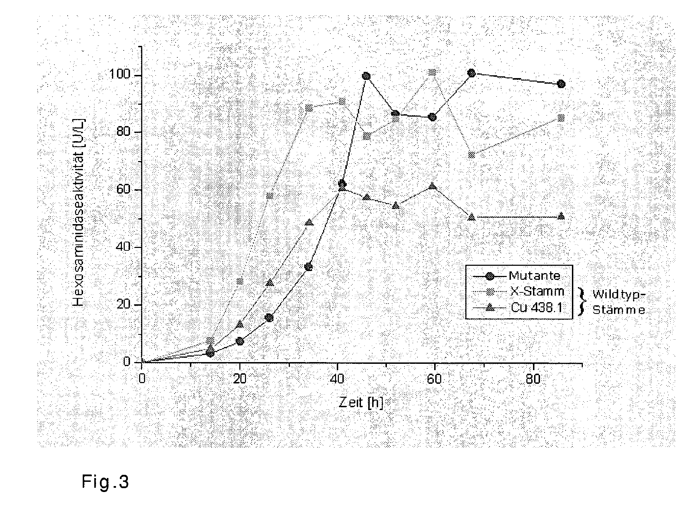

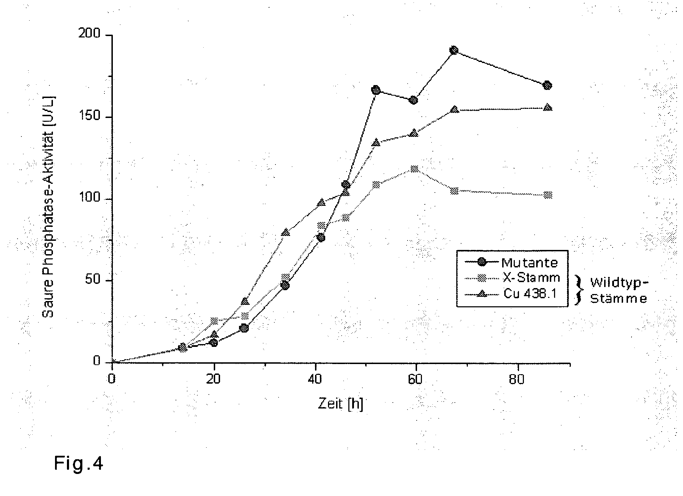

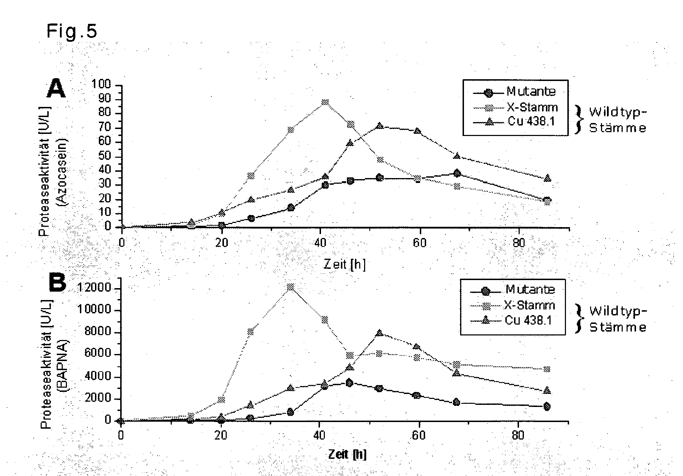

In Abb. 3 und Abb. 4 sind Sekretionskinetiken einer Mutante im Vergleich zu Wildtyp-Stämmen (Wildtypstamm X und Wildtypstamm CU 438.1) dargestellt. Die Volumenaktivitäten für zwei typische sekretierte lysosomale Enzyme, die beta-Hexosaminidase und die saure Phosphatase, sind bei der Mutante vergleichbar mit den Volumenaktivitäten bei Wildtypen. In Fig. 5 sind Sekretionskinetiken für Protease von einer Mutante und zwei Wildtypen dargestellt. Deutlich wird hier, dass die Mutante während der Kultivierungsdauer erheblich weniger Protease in das umgebende Kulturmedium abgibt, als die Wildtyp-Stämme dies tun.

### Figuren:

Figur 1 zeigt einen schematischer Aufbau des erfindungsgemäßen Fluoreszenz-Sceeningverfahrens.
Figur 2 zeigt einen Röntgenfilm nach Durchführung des Screenings.
Figur 3 zeigt eine Sekretionskinetik mit Volumenaktivitäten der ß-Hexosaminidase im zellfreien Überstand von einer Mutante und den beiden Wildtypstämmen X und Cu 438.1.
Figur 4 zeigt eine Sekretionskinetik mit Volumenaktivitäten der sauren Phosphatase im zellfreien Überstand von einer Mutante und den beiden Wildtypstämmen X und Cu 438.1.
Figur 5 zeigt eine Sekretionskinetik mit Volumenaktivitäten der Proteasen im zellfreiem Überstand von einer Mutante und den beiden Wildtypstämmen X und Cu 438.1.

### Literatur:

BANNO, Y. & NOZAWA, Y. (1982): Changes in particulate-bound protease activity during cold acclimation in Tetrahymena pyriformis; Biochim. Biophys. Acta 719: 20-28
BANNO, Y., YANO, K. & NOZAWA, Y. (1982): Biochemical characterization of secreted proteases during growth in Tetrahymena pyriformis WH-15: Comparison of extracellular with intracellular proteases; J. Protozool. 29: 91-98
BANNO, Y., YANO, K. & NOZAWA, Y. (1983): Purification and characterization of a secreted protease from Tetrahymena pyriformis; Eur. J. Biochem.
CASSIDY-HANLEY, D., BOWEN, J., LEE, J. H., COLE, E., VERPLANKT, L: A., GAERTIG, J., GOROWSKY, M. A. & BRUNS, P. J. (1997): Germline and somatic transformation of mating Tetrahymena thermophila by particle bombardment; Genetics 146: 135-147
COLE, E. S. & BRUNS, P. J. (1992): Uniparental Cytogamy: A novel method for bringing micronuclear mutations of Tetrahymena into homozygous macronuclear expression with precocious sexual matury; Genetics 132: 1017-1031
CRUEGER, W. & CRUEGER, A. (1989): Biotechnologie - Lehrbuch der angewandten Mikrobiologie- 3. Aufl.: 3. Stammentwicklung: 7-54; Oldenburg Verlag GmbH, München
GAERTIG, J., & GOROVSKY, M. A. (1992): Efficient mass transformation of Tetrahymena thermophila by electroporation of conjugants; Proc. Natl. Acad. Sci. USA 89: 9196-9200
GAERTIG, J., GU, L., HAI, B. & GOROVSKY, M. A. (1994): High frequent vector-mediated transformation and gene replacement in Tetrahymena; Nucleic Acids Res. 22: 5391-5398
HARTMANN, M., GUBERMANN,A., FLORIN-CHRISTENSEN, M. & TIEDTKE, A. (2000): Screening for and characterization of phospholipase A1 hypersecretory mutants of Tetrahymena thermophila; Appl. Microbiol. Biotechnol. 54: 390-396
HÜNSELER, P., SCHEIDGEN-KLEYBOLDT, G. & TIEDTKE, A. (1987): Isolation and characterization of a mutant of Tetrahymena thermophila blocked in secretion of lysosomal enzymes; J. Cell Sci. 88: 47-55
KIY, T. & TIEDTKE, A. (1992): Continous high-cell-density fermentation of the ciliated protozoon Tetrahymena in a perfused bioreactor; Appl. Micobiol. Biotechnol. 38: 141-146
KIY, T. (1993): Fermentation von Ciliaten zur Produktion biogene Wertstoffe; Inaugural-Dissertation zur Erlangung des Doktorgrades; Institut für Allgemeine Zoologie und Genetik, Westfälische Wilhelms-Universität Münster PAECH, C., CHRISTIANSON, T., & MAURER, K.-H. (1993): Zymogram of proteases made with developed film from non-denaturing polyacrylamide gels after elctrophoresis; Analyt. Biochem. 208: 249-254
TANIGUCHI, T., MIZUOCHI, T., BANNO, Y., NOZAWA, Y. & KOBATA, A. (1995): Carbohydrates of lysosomal enzymes secreted by Tetrahymena pyriformis; J. Biol. Chem. 260 (26): 13941-13946
TONDRAVI, M. M., & YAO, M.-C. (1986): Transformation of Tetrahymena thermophila by microinjection of ribosomal RNA genes; Proc. Natl. Acad. Sci. USA83: 4369-4373
YU, G. L., BRADLEY, ATTARDI, L. D. & BLACKBURN, E. H. (1990): In vitro alteration of telomere sequences and senescence caused by mutated Tetrahymena telomerase RNAs; Nature 344: 126-132

## Patentansprüche

1. Verfahren zur Identifizierung Protease-sekretionsdefizienter Stämme eines Mikroorganismus,
• wobei Mutanten des Mikroorganismus erzeugt werden,
• in den Vertiefungen einer Mikrotiterplatte ein Gel erzeugt wird,
• in jede der Vertiefungen auf das Gel eine Mutante des Mikroorganismus gegeben wird,
• inkubiert wird unter Bedingungen, unter denen die Mutanten des Mikroorganismus Proteine sekretieren,
• die Mutanten des Mikroorganismus vom Gel abgetrennt werden,
wobei in dem Gel mindestens ein Substrat für mindestens eine Sekretionsprotease des Mikroorganismus enthalten ist und/oder das Gel selbst das Substrat ist und/oder das Substrat später zugesetzt wird und mindestens teilweise in das Gel hineindiffundiert,
und die Proteaseaktivität auf das Substrat gemessen wird.

2. Verfahren nach Anspruch 1, wobei der Mikroorganismus ausgewählt ist aus der Gruppe bestehend aus Ciliaten, insbesondere *Tetrahymena,* Hefen, insbesondere *Pichia pastoris,* einzelliger Algen, insbesondere *Chlamydomonas, Ulva* und *Euglena,* und Prokaryonten, insbesondere *Bacillus* und *Escherichia.*

3. Verfahren nach Anspruch 1 oder 2, wobei die Mutanten des Mikroorganismus erzeugt werden durch Zufallsmutagenese oder gezielte Mutagenese.

4. Verfahren nach Anspruch 1 oder 2, wobei die Mutanten des Mikroorganismus erzeugt werden durch Uniparentale Cytogamie (UPC).

5. Verfahren nach einem der Ansprüche 1 bis 4, wobei die Inkubation der Mutanten des Mikroorganismus mit dem Gel in einem Nährmedium erfolgt.

6. Verfahren nach Anspruch 5, wobei das Nährmedium Magermilchpulver, Proteose Peptone, Hefeextrakt, Sojabohnen-Peptone, Ferrous Sulphate/Chelate Solution und/oder Glucosemonohydrat enthält oder ein synthetisches Medium ist.

7. Verfahren einem der Ansprüche 1 bis 6, wobei das Gel ein Gelatine-, Agarose-, Agar- und/oder Polyacrylamidgel ist.

8. Verfahren nach einem der Ansprüche 1 bis 7, wobei das Substrat für die Protease ausgewählt ist aus der Gruppe bestehend aus Casein, Derivaten des Casein, fluoreszent markiertem Casein und/oder Gelatine.

9. Verfahren nach einem der Ansprüche 1 bis 8, wobei die Inkubation erfolgt unter Bedingungen, bei denen der Mikroorganismus Proteasen sekretiert und sekretierte Proteasen in das Gel diffundieren können.

10. Verfahren nach einem der Ansprüche 1 bis 9, wobei der Mikroorganismus von dem Gel abgetrennt wird durch abgießen, abpipettieren, absaugen und/oder abwaschen.

11. Verfahren nach einem der Ansprüche 1 bis 10, wobei die Proteolyse des Substrats ein Fluoreszenzsignal oder Absorptionssignal erzeugt, dessen Intensität gemessen wird und anhand des Ergebnisses die Proteaseaktivität bestimmt wird.

12. Verfahren nach einem der Ansprüche 1 bis 11, wobei das Verfahren automatisiert durchgeführt wird.

## Claims

1. A method for identifying protease secretion deficient strains of a microorganism, wherein:
• mutants of the microorganism are produced;
• a gel is produced in the wells of a microtitration plate;
• a mutant of the microorganism is added to each of the wells onto the gel;
• incubation is effected under conditions under which the mutants of the microorganism secrete proteins;
• said mutants of the microorganism are separated from the gel;
wherein at least one substrate for at least one secretion protease of the microorganism is contained in said gel and/or said gel itself is the substrate and/or the substrate is added later and will at least in part diffuse into the gel;
and the protease activity that has acted on the substrate is measured.

2. The method according to claim 1, wherein said microorganism is selected from the group consisting of ciliates, especially *Tetrahymena,* yeasts, especially *Pichia pastoris,* monocellular algae, especially *Chlamydomonas, Ulva* and *Euglena,* and prokaryotes, especially *Bacillus* and *Escherichia.*

3. The method according to claim 1 or 2, wherein said mutants of the microorganism are produced by random mutagenesis or directed mutagenesis.

4. The method according to claim 1 or 2, wherein said mutants of the microorganism are produced by uniparental cytogamy (UPC).

5. The method according to any of claims 1 to 4, wherein said incubation of the mutants of the microorganism with the gel is effected in a nutrient medium.

6. The method according to claim 5, wherein said nutrient medium contains skim milk powder, proteose peptones, yeast extract, soybean peptones, ferrous sulfate/chelate solution and/or glucose monohydrate or is a synthetic medium.

7. The method according to any of claims 1 to 6, wherein said gel is a gelatin, agarose, agar and/or polyacrylamide gel.

8. The method according to any of claims 1 to 7, wherein said substrate for the protease is selected from the group consisting of casein, derivatives of casein, fluorescence-labeled casein and/or gelatin.

9. The method according to any of claims 1 to 8, wherein said incubation is effected under conditions under which said microorganism secretes proteases, and secreted proteases can diffuse into the gel.

10. The method according to any of claims 1 to 9, wherein said microorganism is separated from the gel by pouring (decanting), pipetting, sucking and/or washing.

11. The method according to any of claims 1 to 10, wherein the proteolysis of the substrate produces a fluorescence signal or absorption signal whose intensity is measured, and the protease activity is determined from the result.

12. The method according to any of claims 1 to 11, wherein said method is performed in an automated format.

## Revendications

1. Procédé d'identification de souches d'un microorganisme qui présentent une déficience de sécrétion de protéase,
• des mutants du microorganisme étant produits,
• un gel étant produit dans les creux d'une plaque de microtitration,
• un mutant du microorganisme étant donné dans chacun des creux sur le gel,
• et étant incubé dans des conditions dans lesquelles les mutants du microorganisme sécrètent des protéines,
• les mutants du microorganisme étant séparés du gel, un substrat pour au moins une protéase sécrétée du microorganisme étant contenu dans le gel, et/ou le gel lui-même étant le substrat, et/ou le substrat étant ajouté ultérieurement et diffusant au moins partiellement dans le gel,
et l'activité protéasique sur le substrat étant mesurée.

2. Procédé selon la revendication 1, le microorganisme étant sélectionné dans le groupe composé de ciliates, en particulier *Tetrahymena,* de levures, en particulier *Pichia pastoris,* d'algues unicellulaires, en particulier *Chlamydomonas, Ulva* et *Euglena,* et de procaryotes, en particulier *Bacillus* et *Escherichia.*

3. Procédé selon la revendication 1 ou 2, les mutants du microorganisme étant produits par mutagenèse aléatoire ou par mutagenèse ciblée.

4. Procédé selon la revendication 1 ou 2, les mutants du microorganisme étant produits par cytogamie uniparentale (UPC).

5. Procédé selon l'une des revendications 1 à 4, l'incubation des mutants du microorganisme avec le gel s'effectuant dans un milieu de culture.

6. Procédé selon la revendication 5, le milieu de culture contenant de la poudre de lait maigre, de la protéose-peptone, de l'extrait de levure, de la peptone de fève de soja, une solution de sulfate/chélate ferreux et/ou du glucose monohydrate, ou bien étant un milieu de synthèse.

7. Procédé selon l'une des revendications 1 à 6, le gel étant un gel de gélatine, d'agarose, d'agar-agar et/ou de poly(acrylamide).

8. Procédé selon l'une des revendications 1 à 7, le substrat pour la protéase étant choisi dans le groupe composé par la caséine, les dérivés de la caséine, la caséine marquée par fluorescence et/ou la gélatine.

9. Procédé selon l'une des revendications 1 à 8, l'incubation s'effectuant dans des conditions dans lesquelles le microorganisme sécrète des protéases, et les protéases sécrétées peuvent diffuser dans le gel.

10. Procédé selon l'une des revendications 1 à 9, le microorganisme étant séparé du gel par décantation, pipetage, aspiration et/ou lavage.

11. Procédé selon l'une des revendications 1 à 10, la protéolyse du substrat produisant un signal fluorescent ou un signal d'absorption dont l'intensité est mesurée, et l'activité protéasique étant déterminée à l'aide du résultat.

12. Procédé selon l'une des revendications 1 à 11, le procédé étant réalisé de façon automatisée.
